# EUROPEAN PATENT APPLICATION

(11) **EP 1 849 793 A1**
(43) Date of publication of application: **31.10.2007**
(21) Application number: 05844822.6
(22) Date of filing: 28.12.2005
(51) Int. Cl.: C07H 3/02, C07H 3/08, C07C 317/18, C07C 323/12

(54) **PROCESS FOR PRODUCING CARBON-DIMINISHED ALDOSE COMPOUND**

(30) Priority: 28.12.2004 JP 2004379231
(71) Applicant: Asubio Pharma Co., Ltd., Minato-ku Tokyo (JP)
(72) Inventor: OKITSU, Mitsuhito, Asubio Pharma Co., Ltd., Mishima-gun, Osaka 618-8513 (JP); KAMEI, Katsuhide, Asubio Pharma Co., Ltd., Mishima-gun, Osaka 618-8513 (JP)
(74) Representative: Albrecht, Thomas
(86) International application number: PCT/JP2005/024049
(87) International publication number: WO 2006/070862

(57) **Abstract**

The present invention provides a process capable of industrially producing 5-deoxy-L-arabinose, important as a raw material for the production of sapropterin useful as a therapeutic agent for atypical hyperphenylalaninemia, satisfactorily efficiently even with a simple production apparatus. Provided is the process for producing 5-deoxy-L-arabinose **characterized by** including: reacting L-rhamnose with a C₁₁₋₁₆ straight chain alkyl mercaptan compound in the presence of an acid catalyst to prepare L-rhamnose dialkylmercaptal; subjecting then the obtained compound to an oxidation reaction to prepare a sulfonyl derivative; and subjecting then the sulfonyl derivative to a carbon-reduction reaction to prepare 5-deoxy-L-arabinose. The present production process can also be applied to a process for producing compounds obtained by removing one carbon atom from other aldol compounds, and thus the present invention provides a general process for producing compounds obtained by reducing the number of carbon atoms from aldose compounds.

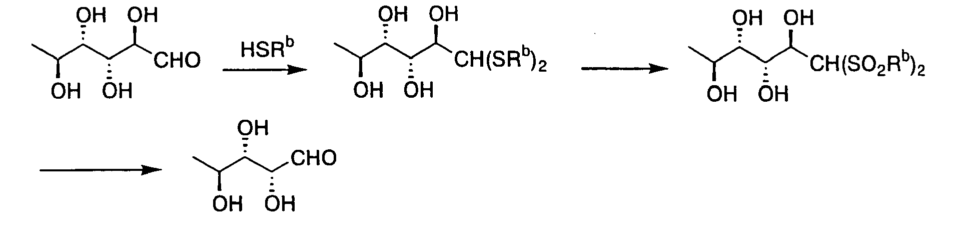

## Description

### TECHNICAL FIELD

The present invention relates to a process for producing a compound obtained by removing one carbon atom from an aldose compound, in particular, to a new, industrially excellent process for producing 5-deoxy-L-arabinose useful as a synthetic intermediate for (R)-2-amino-6-[(1R,2S)-1,2-dihydroxypropyl]-5,6,7,8-tetrahydro-4(3H)-pteridinone (the dihydrochloride thereof has a generic name: sapropterin hydrochloride) being clinically used as a therapeutic agent for atypical hyperphenylalaninemia.

### BACKGROUND ART

A compound represented by the following formula (A):

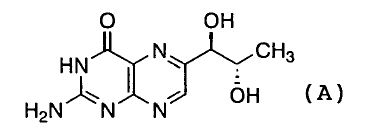

namely, L-erythro-biopterin is one of the pteridines widely occurring in nature, which is found in microorganisms, insects, algae, amphibia, mammals and the like (Non-patent Document 1). The tetrahydro derivative of this compound represented by the following formula (B):

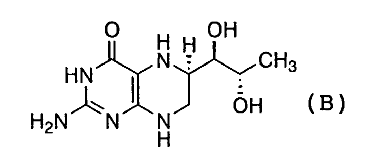

namely, (R)-2-amino-6-[(1R,2S)-1,2-dihydroxypropyl]-5,6,7,8-tetrahydro-4(3H)-pteridinone (hereinafter, abbreviated as R-THBP, as the case may be) functions as a coenzyme essential for biological hydroxylation reaction and oxygenase reaction; thus the dihydrochloride thereof has already been approved and put on the market, in some countries inclusive of Japan, under the commercial name of Biopten (Trade Mark) (generic name: sapropterin hydrochloride) as a sole effective therapeutic agent for atypical hyperphenylalaninemia known as an intractable disorder.
On the other hand, the above-mentioned tetrahydro derivative has a function as a coenzyme for the NO synthesis enzyme (nitrogen monoxide synthesis enzyme), and accordingly, the possibility of this derivative as the therapeutic agents for various disorders have recently come to be reported.

However, although several processes have been reported on the process for producing R-THBP, practically excellent one among these processes is only a production process in which R-THBP represented by formula (B) is produced from L-erythro-biopterin represented by formula (A) (Patent Document 1). Accordingly, the essence for the industrial process for producing R-THBP represented by formula (B) resides in how efficiently L-erythro-biopterin, to be a starting compound, represented by formula (A) can be produced.

Additionally, as the process for synthesizing L-erythro-biopterin represented by formula (A), a process has hitherto been known in which some appropriate saccharide intermediate is condensed with triaminouracil, namely, 2,4,5-triamino-6-hydroxypyrimidine or compounds related thereto.

For example, there have been reported a process due to J. M. Andrews et al. in which an α-amino ketone compound derived from 5-deoxyarabinose and 2-amimo-4-chloro-3-nitro-6-hydroxypyrimidine are condensed with each other (Non-patent document 2), and processes developed by further improving this process (Non-patent Documents 3 and 4); however, any of these processes is insufficient both in optical purity and chemical purity to be unsatisfactory as industrial production processes.

Thereafter, J. Welustock et al. (Patent Document 2) and E. C. Taylor et al. (Non-patent Document 5) proposed a more practical process higher in optical selectivity. In this process, as shown by the following reaction scheme:

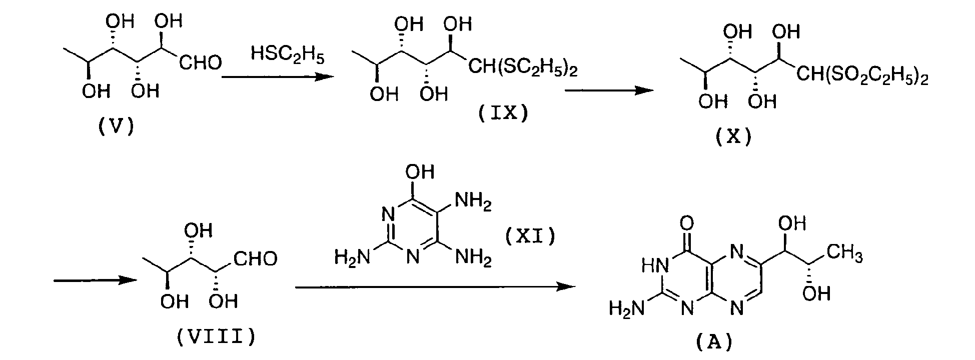

L-rhamnose (V) is used as a starting material and reacted with ethanethiol (C₂H₅SH) to synthesize L-rhamnose diethylmercaptal represented by formula (IX); L-rhamnose diethylmercaptal is successively converted into a disulfone derivative represented by formula (X) through an oxidation reaction; the disulfone derivative is subjected to carbon elimination to prepare 5-deoxy-L-arabinose, represented by formula (VIII), in which one carbon atom is removed from L-rhamnose; and 5-deoxy-L-arabinose is condensed with triaminouracil represented by formula (XI), namely, 2,4,5-triamino-6-hydroxypyrimidine to yield L-erythro-biopterin represented by formula (A).

Thereafter, this process has been further improved by Schircks et al. (Non-patent Document 6); the key point of this synthetic process resides in how industrially efficiently 5-deoxy-L-arabinose, important as a synthesis intermediate, represented by formula (VIII) can be produced, namely, how industrially efficiently 5-deoxy-L-arabinose obtained by removing one carbon atom from L-rhamnose can be produced.

At present, the above-mentioned process is put to practical use as a sole industrial production process of R-THBP; however, it is to be noted that this process uses ethanethiol, namely, ethyl mercaptan that is a compound having a boiling point as low as 35°C and emits intolerable malodor, in the step of conversion from L-rhamnose represented by formula (V) to L-rhamnose diethylmercaptal represented by formula (IX). Accordingly, this process not only degrades the working environment but also offers a cause of air pollution in the vicinity of the work area, to preclude production with common production facilities and to inevitably require a special odor prevention unit and a special deodorization unit for the purpose of preventing environmental pollution.

Thus, in order to enable industrial scale production of R-THBP to be carried out more inexpensively, as affairs now stand, it is desired to establish an alternative process which can efficiently produce 5-deoxy-L-arabinose represented by formula (VIII) essential for such production step as described above, with a common simple apparatus.

Patent Document 1: U.S. Patent No. 4713453
Patent Document 2: U.S. Patent No. 3505329
Non-patent Document 1: E. L. Patterson et al.; J. Am. Chem. Soc., 78: 5868 (1956)
Non-patent Document 2: J. M. Andrews et al.; J. Chem. Soc., 928 (1969)
Non-patent Document 3: M. Viscontini et al.; Helv. Chem. Acta., 55: 570 (1972)
Non-patent Document 4: H. Rembold; Chem. Ber., 96: 1395 (1963)
Non-patent Document 5: E. C. Taylor et al.; J. Am. Chem. Soc., 98: 2301 (1979)
Non-patent Document 6: B. Schircks et al.; Helv. Chem. Acta., 68: 1639 (1985)
Non-patent Document 7: M. Node et al.; Tetrahedron Lett., 42: 9207 (2001)

Accordingly, if a simple process for producing a carbon-reduced compound of L-rhamnose, needing no such a special odor prevention unit and no such a special deodorization unit can be found, it means that such a process can be applied to a process for producing a compound obtained by reducing by one the number of carbon atoms not only from L-rhamnose represented by formula (V) but from a wide variety of aldose compounds, namely, monosaccharides each having an aldehyde group.

### DISCLOSURE OF THE INVENTION

### PROBLEMS TO BE SOLVED BY THE INVENTION

Accordingly, in view of the present circumstances, the present invention takes as its problem the provision of a simple process for producing a compound obtained by removing one carbon atom from an aldose compound, needing no special odor prevention unit and no special deodorization unit, in particular, a process capable of producing 5-deoxy-L-arabinose, represented by formula (VIII), important as a raw material for producing (R)-2-amino-6-[(1R,2S)-1,2-dihydroxypropyl]-5,6,7,8-tetrahydro-4(3H)-pteridinone (R-THBP), represented by formula (B), useful as a therapeutic agent for atypical phenylalaninemia, wherein malodor generation is conventionally avoided, and the production can be carried out efficiently in an industrial scale even with a simple production apparatus.

### MEANS TO SOLVE THE INVENTION

A most serious problem in the prior production of 5-deoxy-L-arabinose represented by formula (VIII) is the use of ethanethiol having intolerable malodor. If an alternative production process is found in which an odorless thiol compound having a chemical reactivity similar to that of ethanethiol and capable of being used in an industrial scale is used instead of using this ethanethiol, there can be established an inexpensive process for producing targeted 5-deoxy-L-arabinose represented by formula (VIII) which process does not need any improvement for work environment, prevention of air pollution and a special odor prevention or deodorization unit.

Although those skilled in the art have shared a semi-commonsense recognition that "thiols have malodor," Node et al. have reported odorless thiols to overthrow such a concept (Non-patent Document 7). According to this research, it is reported that thiols having malodor are straight chain alkyl mercaptan compounds having 10 or less carbon atoms, and on the other hand, C₁₁₋₁₆ straight chain alkyl mercaptan compounds each have a melting point of 200°C or higher, lack volatility at normal pressures, and little smell on the basis of the six-level odor intensity indication method.

The present inventors have conceived that the use of the C₁₁₋₁₆ straight chain alkyl mercaptan compounds leads to a new extremely effective process for producing the compounds, typified by 5-deoxy-L-arabinose represented by formula (VIII), obtained by removing one carbon atom from aldose compounds, and results in solution of many problems caused by use of ethanethiol. Accordingly, the present inventors have investigated commercially available long chain alkyl thiol compounds, and have found that C₁₁₋₁₆ straight chain alkyl mercaptan compounds are available and slightly smell, and have conceived that excellent among these compounds is dodecanethiol having 12 carbon atoms because this compound was identified to be odorless even with a smell analyzer incorporating a semiconductor sensor, manufactured by Shimadzu Corporation (Fragrance & Flavor Analyzer; FF-1).

Absolutely no reactions between the C₁₁₋₁₆ straight chain alkyl mercaptan compounds and the aldose compounds have been known except for the reaction involving xylose, and there have been known absolutely no series of processes for producing the compounds obtained by removing one carbon atom from aldose compounds by using the C₁₁₋₁₆ alkyl mercaptan compounds.
Accordingly, the present inventors have studied various reaction conditions by using these odorless mercaptan compounds, and consequently have found that 5-deoxy-L-arabinose represented by formula (VIII) can be efficiently produced with a simple apparatus and simple facilities.

Consequently, the present invention provides a process for producing a compound obtained by removing one carbon atom from an aldose compound, as a basic aspect of the present invention, and specifically, a process for producing a compound (I) obtained by removing one carbon atom from an aldose compound, the process being characterized by including:

reacting an aldose compound represented by the following formula (IV) with a C₁₁₋₁₆ straight chain alkyl mercaptan compound in the presence of an acid catalyst: 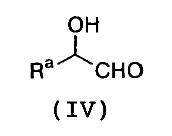 wherein R^{a} represents the residual group in the aldose compound, to prepare a dialkylmercaptal compound represented by the following formula (III):

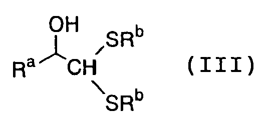

wherein R^{b} represents a C₁₁₋₁₆ straight chain alkyl group, and R^{a} is defined as the same as above; subjecting then the obtained compound represented by formula (III) to an oxidation reaction to prepare a disulfonyl derivative represented by the following formula (II):

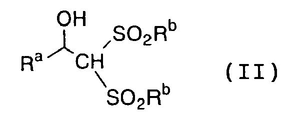

wherein R^{a} and R^{b} are defined as the same as above; and treating in base the obtained disulfonyl derivative represented by formula (II) to prepare a compound represented by the following formula (I):

R^{a}-CHO (I)

wherein R^{a} is defined as the same as above.

Specifically, provided is a production process wherein the aldose compound represented by formula (IV) is D-glucose, L-rhamnose, D-mannose, D-galactose, L-arabinose, D-xylose or D-lyxose, in particular, a production process wherein the aldose compound is L-rhamnose.

More specifically, the present invention is a process for producing 5-deoxy-L-arabinose represented by formula (VIII) important as a raw material for production of R-THBP, and in more detail, a process for producing 5-deoxy-L-arabinose obtained by removing one carbon atom from L-rhamnose represented by formula (V).

In other words, more specifically, provided is a process for producing 5-deoxy-L-arabinose, the process being characterized by including:

reacting L-rhamnose represented by the following formula (V) 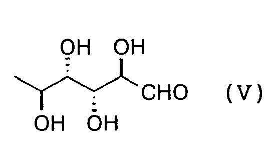 with a C₁₁₋₁₆ straight chain alkyl mercaptan compound in the presence of an acid catalyst to prepare a dialkylmercaptal compound represented by the following formula (VI):

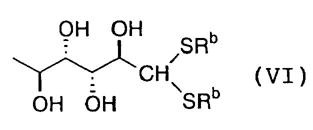

wherein R^{b} represents a C₁₁₋₁₆ straight chain alkyl group; then, subjecting the obtained compound represented by formula (VI) to an oxidation reaction to prepare a disulfonyl derivative represented by the following formula (VII):

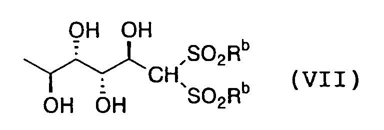

wherein R^{b} is defined as the same as above; and treating in base the obtained disulfonyl derivative represented by formula (VII) to prepare 5-deoxy-L-arabinose represented by the following formula (VIII):

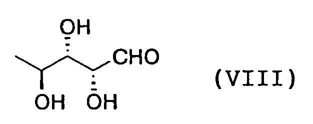

In the above-mentioned series of production processes, the reactivity of the C₁₁₋₁₆ straight chain alkyl mercaptan compound is satisfactory so as to easily react with the aldose compound similarly to ethanethiol; on the other hand, the C₁₁₋₁₆ dialkyl sulfonylmethane generated in the final step in an amount of 1 equivalent is large in molecular weight, is precipitated as crystals with the progress of the reaction and can thereby be easily removed from the reaction mixture without involving any cumbersome purification step based on silica gel column chromatography; the whole steps do not suffer from malodor so as to drastically improve the work environment.

Additionally, the present invention provides as another aspect thereof a new important intermediate in the above-mentioned production processes, namely, a dialkylmercaptal compound represented by the following formula (VI):

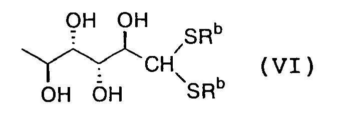

wherein R^{b} represents a C₁₁₋₁₆ straight chain alkyl group.

Further, the present invention provides another new intermediate, namely, a disulfonyl derivative represented by the following formula (VII):

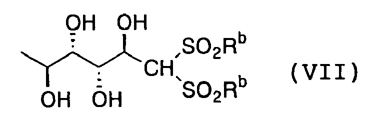

wherein R^{b} represents a C₁₁₋₁₆ straight chain alkyl group.

### EFFECT OF THE INVENTION

According to the production process provided by the present invention, a compound obtained by removing one carbon atom from an aldose compound can be efficiently produced without generating intolerable malodor. In particular, the production process of the present invention has very high industrial applicability because it can efficiently produce a targeted compound obtained by removing one carbon atom from an aldose compound by using a common production apparatus without needing the use of any special production apparatus incorporating a special odor prevention unit or a special deodorization unit.

Particularly, among others, 5-deoxy-L-arabinose represented by formula (VIII) important as a raw material for production of R-THBP can be efficiently produced with a common production apparatus without using any special production apparatus incorporating a special odor prevention unit or a special deodorization unit; hence work efficiency improvement can be attained, accordingly R-THBP useful as a therapeutic agent for atypical hyperphenylalaninemia can be inexpensively provided, and thus, the medical contribution of the present invention is significant.

### BEST MODE FOR CARRYING OUT THE INVENTION

The process for producing a compound obtained by removing one carbon atom from an aldose compound, provided by the present invention as a first aspect thereof, may be summarized as follows in terms of a specific chemical reaction formula.

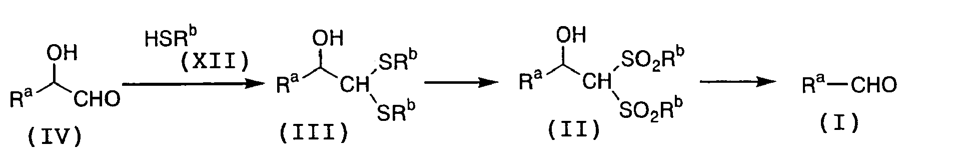

wherein R^{a} represents the residual group in the aldose compound, and R^{b} represents a C₁₁₋₁₆ straight chain alkyl group.

In this case, examples of the aldol compound represented by formula (IV) include monosaccharides each having an aldehyde group, specifically, D-glucose, L-rhamnose, D-mannose, D-galactose, L-arabinose, D-xylose and D-lyxose. The chemical structural formulas of these compounds are as follows:

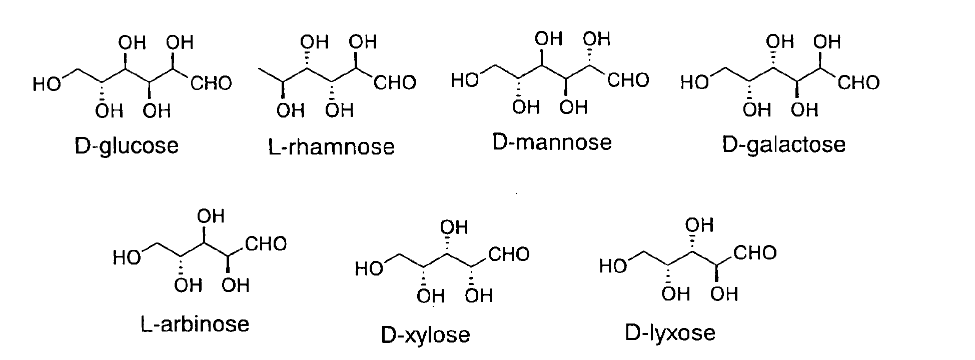

Therefore, the residual group of the aldose compound represented by R^{a} in the above chemical reaction formula means any of the residual groups of these aldose compounds.

The process, provided by the present invention, for producing the compound represented by formula (I) based on the reduction of the number of carbon atoms from an aldose compound represented by formula (IV) is specifically a production process in which the aldose compound (IV) is reacted with a C₁₁₋₁₆ straight chain alkyl mercaptan compound (XII) in the presence of an acid catalyst to prepare a dialkylmercaptal compound represented by formula (III), then the obtained compound (III) is subjected to an oxidation reaction to prepare a disulfone derivative represented by formula (II), and the disulfone derivative (II) is treated in base for reducing the number of carbon atoms to produce the objective compound represented by formula (I).

The process of the present invention is characterized in that, in place of ethanethiol having been used in conventional processes for producing the compound represented by formula (I), the C₁₁₋₁₆ straight chain alkyl mercaptan compound represented by formula (XII) is used.
Specific examples of such a C₁₁₋₁₆ straight chain alkyl mercaptan compound represented by formula (XII) include undecanethiol (C₁₁), dodecanethiol (C₁₂), tridecanethiol (C₁₃), tetradecanethiol (C₁₄), pentadecanethiol (C₁₅) and hexadecanethiol (C₁₆). Among these, dodecanethiol having 12 carbon atoms is particularly preferably used.

These thiol compounds are specific in the sense that they have no volatility, little have malodor characteristic of mercaptan compounds, are extremely easy to handle, and require no installation of a special apparatus, as a reaction apparatus to use these compounds, incorporating a special odor prevention unit or a special deodorization unit. In particular, among these mercaptan compounds, dodecanethiol having 12 carbon atoms is a particularly odorless compound, and is a preferable mercaptan compound extremely easy to handle.

By means of the above-mentioned production process, the targeted compound represented by formula (I) can de derived; the process concerned is particularly useful as a process for producing 5-deoxy-L-arabinose represented by formula (VIII) important as a raw material for production of R-THBP by removing one carbon atom from L-rhamnose represented by corresponding formula (V).

The above-mentioned process may be depicted as follows in terms of a chemical reaction scheme:

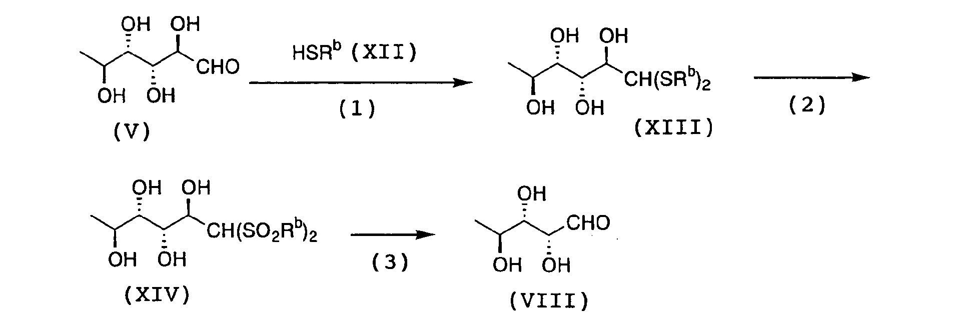

wherein R^{b} represents a C₁₁₋₁₆ straight chain alkyl group, and the numbers in parentheses indicate the individual step numbers.

Accordingly, the present invention is described in detail by describing, as a typical example, the details of the above-mentioned specific production of 5-deoxy-L-arabinose (VIII).

Step 1 is a step in which L-rhamnose represented by formula (V) is reacted with a C₁₁₋₁₆ straight chain alkyl mercaptan compound (XII) in the presence of an acid catalyst to produce a L-rhamnose dialkylmercaptal represented by formula (XIII).
Examples of the acid catalyst to be used in the reaction may include acids used in common thioacetal reaction of mercaptan compounds with aldehyde groups; from the viewpoint of the industrial production process, preferably it is recommended to use hydrochloric acid.

The reaction can be carried out by using of 2 equivalents of mercaptan compound represented by formula (XII) against 1 equivalent of L-rhamnose represented by formula (V), and by stirring in the presence of an acid catalyst in an organic solvent at room temperature for 5 to 20 hours. The organic solvent to be used is not particularly limited unless the organic solvent is involved in the reaction concerned; the following solvents which are capable of dissolving both compounds represented by formula (V) and formula (XII) are preferable: cyclic ethers such as dioxane and tetrahydrofuran; secondary and tertiary alcohols such as isopropanol and t-butanol; and acetic acid and the like. Preferably it is recommended to use dioxane.

In the step concerned, L-rhamnose dialkylmercaptal represented by formula (XIII) as a reaction product has such advantages that L-rhamnose dialkylmercaptal is usually precipitated as crystals in the reaction system, and the crystals are collected, washed appropriately with an organic solvent, then dried, and can be used in the next step without further purification.

Step 2 is a step in which the thus obtained L-rhamnose dialkylmercaptal represented by formula (XIII) is oxidized to be converted to a disulfonyl derivative represented by formula (XIV). Usually, the reaction can be carried out by dissolving L-rhamnose dialkylmercaptal represented by formula (XIII) in an appropriate solvent, adding an oxidant to the reaction solution thus obtained, and stirring the reaction mixture at 0°C to 80°C, preferably, at room temperature.

Examples of the oxidant to be used may include hydrogen peroxide, peracetic acid, m-chloroperbenzoic acid and oxone; in consideration of industrial production processes, it is preferable to use hydrogen peroxide. The concentration of the hydrogen peroxide solution is not particularly limited; hydrogen peroxide is a particularly excellent oxidant in the sense that a common commercially available 30% hydrogen peroxide solution can be used. The solvent to be used is not particularly limited; the solvent capable of dissolving the compound represented by formula (XIII) is preferable, and acetic acid is particularly preferable.

The reaction time is also not particularly limited; when the reaction is allowed to proceed at room temperature, it is preferable to carry out the reaction for approximately 10 to 25 hours so as to complete the concerned oxidation reaction. After the completion of the reaction, the objective disulfonyl derivative represented by formula (XIV) can be obtained as crystals by the treatment heretofore known per se such as extraction, washing or concentration with an organic solvent.

Step 3 is a step in which the disulfonyl derivative obtained in Step 2 represented by formula (XIV) is subjected to carbon elimination (carbon reduction) to produce one of the target compounds of the present invention, namely, 5-deoxy-L-arabinose represented by formula (VIII). The carbon elimination concerned is carried out by dissolving the compound represented by formula (XIV) in an appropriate organic solvent to be subjected to treatment in base; such treatment is carried out by adding aqueous ammonia having an appropriate concentration to the reaction mixture thus obtained and by stirring the reaction mixture.

Preferable as the solvent to be used is a solvent which dissolves the compound represented by formula (XIV) and is miscible with aqueous ammonia; for example, by using dioxane, the treatment with base can be preferably carried out. The concentration of the aqueous ammonia to be added cannot be unconditionally limited; however, from the viewpoint of an industrial production process, the treating method in base may be preferably carried out by means of using common commercially available 28% aqueous ammonia as a base.

The reaction temperature and the reaction time of the carbon elimination cannot be unconditionally limited; however, by adding the stirring treatment with stirring, the carbon elimination can be completed by treating at room temperature, for example, for approximately 0.5 to 5 hours.

In Step concerned, as a result of the carbon elimination treatment, dialkylsulfonylmethane represented by the following formula (XV) is obtained as crystals from the reaction mixture:

CH₂(SO₂R^{b})₂ (XV)

wherein R^{b} represents a C₁₁₋₁₆ straight chain alkyl group. Consequently, with respect to the reaction treatment, such dialkylsulfonylmethane represented by formula (XV) is removed by filtration treatment or the like, and the filtrate thus obtained is subjected, after concentration of the reaction mixture, to a treatment heretofore known per se such as extraction, washing or concentration with an organic solvent. Thus, the objective compound represented by formula (VIII), namely, 5-deoxy-L-arabinose can be obtained as the residue in a form of an oily product.

From above Steps, the objective compound of the present invention, namely, 5-deoxy-L-arabinose represented by formula (VIII) can be industrially efficiently produced by avoiding the conventional malodor generation, with a simple production apparatus; obtained 5-deoxy-L-arabinose represented by formula (VIII) is, as it is, subjected to condensation reaction with triaminouracil represented by formula (XI), namely, 2,4,5-triamino-6-hydroxypyrimidine to be converted into L-erythro-biopterin represented by formula (A); L-erythro-biopterin is further subjected to reduction afford (R)-2-amino-6-[(1R,2S)-1,2-dihydroxypropyl]-5,6,7,8-tetrahydro-4(3H)-pteridinone (R-THBP) represented by formula (B) which is an effective therapeutic agent for atypical hyperpheylalaninemia known as an intractable disorder.

The production process of the present invention has been described with L-rhamnose represented by specific formula (V) to be an aldose compound represented by formula (IV) as the starting compound; however, the production process of the present invention can also be embodied by using as another aldose compound D-glucose, D-mannose, D-galactose, L-arabinose, D-xylose or D-lyxose, on the basis of a similar process in conformity with the above-mentioned process.

### EXAMPLES

Hereinafter, the present invention is described in more detail with reference to Examples, but the present invention is not limited to these Examples.

Example 1: Production of L-Rhamnose Didodecylmercaptal (XVI)

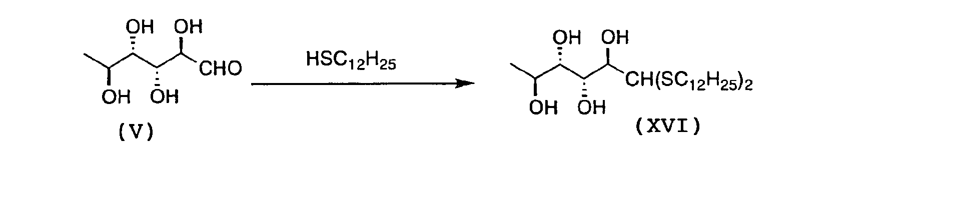

In 20 mL of dioxane, 4.00 g (22 mmol) of L-rhamnose (V) hydrate was dissolved by heating; the solution thus obtained was added dropwise in a 4-N hydrochloric acid/dioxane solution of 8.89 g (44 mmol) of n-dodecanethiol at room temperature with stirring. On completion of dropwise addition, the reaction solution was transparent, and white crystals were precipitated with the progress of the reaction. The reaction solution was stirred at room temperature for 12 hours, and the precipitated white crystals were filtered off, washed with ether and then dried to yield 9.00 g (yield: 75%) of L-rhamnose didodecylmercaptal (XVI) as colorless crystals. This product can be used without further purification in the subsequent reaction; however, the product was recrystallized from dioxane, and thus was able to be obtained as colorless needles.

Melting point: 111 to 112°C
IR (cm⁻¹) : 2917, 2850, 1467, 1064, 972, 894, 721, 613, 507, 422.
NMR (DMSO-D₆/ppm): 4.88(1H, d), 4.41(1H, d), 4.17(1H, s), 4.13(1H, m), 4.05(1H, d), 3.79(1H, m), 3.56(1H, m), 3.29(1H, m), 2.60(4H, m), 1,24(32H, m), 1.12(3H, d), 0,85 (6H, t).

Example 2: Production of 1,1-Didodecylsulfonyl-1-manno-2,3,4,5-tetrahydrohexane (XVII)

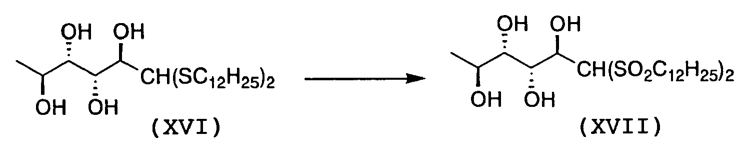

In 100 mL of acetic acid, 4.0g (7.26 mmol) of the L-rhamnose didodecylmercaptal (XVI) obtained in above-mentioned Example 1 was dissolved by heating; to this solution, 40 mL of 30% hydrogen peroxide solution was added dropwise under strong stirring. On completion of addition, the reaction mixture was continuously stirred at room temperature for 18 hours. The reaction mixture with a white precipitate produced therein was diluted with water, and extracted with ethyl acetate. The extract thus obtained was dried and then concentrated. The white precipitate thus obtained was filtered off, and dried to yield 4.02 g (yield: 90%) of 1,1-didodecylsulfonyl-1-manno-2,3,4,5-tetrahydrohexane (XVII). This product can be used without further purification in the subsequent step; however, the product was recrystallized from acetic acid, and thus was able to be obtained as colorless needles.

Melting point: 114-116°C
IR (cm⁻¹): 2918, 2850, 1296, 1117, 1068, 1036, 570, 479.
NMR (CDCl₃/ppm): 4.89(1H, m), 4.74(1H, d), 4.30(1H, m), 4.07(1H, m), 3.71(1H, m), 3.60(4H, m), 3.45(2H, m), 3.40(1H, m), 3.22(1H, m), 1.90(4H, m), 1.45(4H, m), 1.34(3H, d), 1.26(32H, m), 0.88(6H, t).

Example 3: Production of 5-deoxy-L-arabinose (VIII)

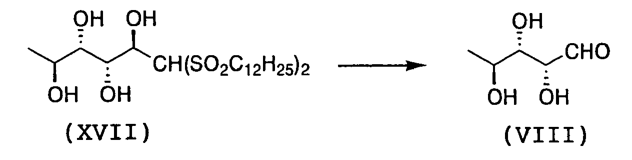

In 10 mL of dioxane, 1.00 g (1.62 mmol) of the 1,1-didodecylsulfonyl-1-manno-2,3,4,5-tetrahydrohexane (XVII) obtained in above-mentioned Example 2 was dissolved, and the solution thus obtained was added under stirring with 5 mL of 28% aqueous ammonia. The thus obtained mixture was stirred at room temperature for 1 hour, and didodecylsulfonylmethane precipitated as a white crystalline precipitate was filtered off to be removed, and the filtrate was concentrated. The residue was added with water and extracted twice with chloroform. The organic layers were combined and concentrated to yield 0.17 g (yield: 80%) of 5-deoxy-L-arabinose (VIII) as a pale yellow oily product.

IR (cm⁻¹) : 3350, 1650, 1560, 1450, 1410, 1380, 1310, 1125, 1060, 1030, 980, 835.
NMR (D₂O/ppm): 5.10(1H, d), 5.07(1H, d), 3.99(1H, m), 3.90(1H, m), 3.58(1H, m), 1.63(3H, d).

In above-mentioned Examples 1 to 3, no malodor characteristic of the mercaptan compound was generated, and the workability of each of these Examples was extremely satisfactory.
It is to be noted that by using mercaptan compounds other than those used in Examples 1 to 3, the operations of Examples 1 to 3 were repeated, and consequently, objective 5-deoxy-L-arabinose (VIII) was able to be obtained.

### INDUSTRIAL APPLICABILITY

As described above, by using the process provided by the present invention, compounds obtained by removing one carbon atom from aldose compounds can be efficiently produced without generating intolerable malodor.
Industrial applicability of such compounds is extremely high because such compounds are used as raw materials for synthesizing various useful compounds, and such compounds can be efficiently produced as targeted compounds obtained by removing one carbon atom from aldose compounds with a common production apparatus, without needing the use of any special production apparatus incorporating a special odor prevention unit or a special deodorization unit.

Additionally, the present invention can efficiently produce, 5-deoxy-L-arabinose, particularly among others, an important compound to be used for production of R-THBP represented by formula (B) that is to be used as a therapeutic agent for atypical hyperphenylalaninemia and a therapeutic agent for R-THBP-reactive phenylketonuria, and useful as a compound having various physiological active effects, with a common production apparatus, without using any special production apparatus incorporating a special odor prevention unit or a special deodorization unit.

Additionally, in processing the reaction concerned, no special reagents are to be used, but common commercially available industrial reagents can be used, and consequently, R-THBP represented by formula (B) can be produced industrially inexpensively, and hence the present invention provides a significant medical contribution.

## Claims

1. A process for producing a compound (I) obtained by removing one carbon atom from an aldose compound, the process being **characterized by** comprising;
reacting an aldose compound represented by the following formula (IV): 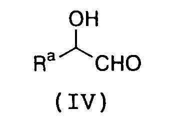 wherein R^{a} represents the residual group in the aldose compound,
with a C₁₁₋₁₆ straight chain alkyl mercaptan compound in the presence of an acid catalyst to prepare a dialkylmercaptal compound represented by the following formula (III): 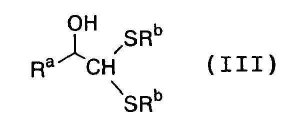 wherein R^{b} represents a C₁₁₋₁₆ straight chain alkyl group, and R^{a} is defined as the same as above;
then, subjecting the obtained compound represented by formula (III) to an oxidation reaction to prepare a disulfonyl derivative represented by the following formula (II): 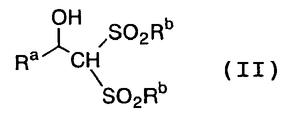 wherein R^{a} and R^{b} are defined as the same as above; and,
treating in base the obtained disulfonyl derivative represented by formula (II) to prepare a compound represented by the following formula (I):
R^{a}-CHO (I)
wherein R^{a} is defined as the same as above.

2. A process for producing a compound (I) obtained by removing one carbon atom from an aldose compound, the process being **characterized by** comprising:
subjecting a dialkylmercaptal compound represented by the following formula (III) to an oxidation reaction: 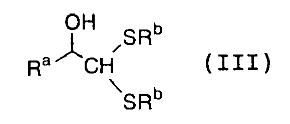
wherein R^{a} represents the residual group in the aldose compound, and R^{b} represents a C₁₁₋₁₆ straight chain alkyl group, to prepare a disulfonyl derivative represented by the following formula (II): 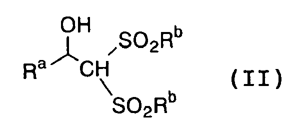 wherein R^{a} and R^{b} are defined as the same as above;
and, treating in base the disulfonyl derivative represented by formula (II) to prepare a compound represented by the following formula (I):
R^{a}-CHO (I)
wherein R^{a} is defined as the same as above.

3. A process for producing a compound (I) obtained by removing one carbon atom from an aldose compound, the process being **characterized by** comprising:
treating in base a disulfonyl derivative represented by the following formula (II): 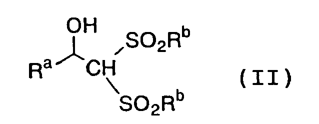
wherein R^{a} represents the residual group in the aldose compound, and R^{b} represents a C₁₁₋₁₆ straight chain alkyl group, to prepare a compound represented by the following formula (I):
R^{a}-CHO (I)
wherein R^{a} is defined as the same as above.

4. The production process according to any one of claims 1 to 3, wherein the residual group in the aldose compound represented by R^{a} in the formulas is a residual group of D-glucose, L-rhamnose, D-mannose, D-galactose, L-arabinose, D-xylose or D-lyxose.

5. The production process according to claim 1 or 2, wherein as the oxidant in the oxidation reaction, hydrogen peroxide, peracetic acid, m-chloroperbenzoic acid or oxone is used.

6. A process for producing 5-deoxy-L-arabinose, the process being **characterized by** comprising:
reacting L-rhamnose represented by the following formula (V) with a C₁₁₋₁₆ straight chain alkyl mercaptan compound in the presence of an acid catalyst: 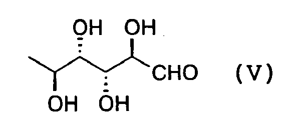 to prepare a dialkylmercaptal compound represented by the following formula (VI): 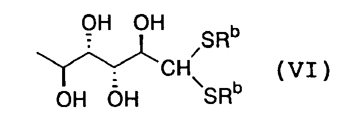
wherein R^{b} represents a C₁₁₋₁₆ straight chain alkyl group;
then, subjecting the obtained compound represented by formula (VI) to an oxidation reaction to prepare a disulfonyl derivative represented by the following formula (VII): 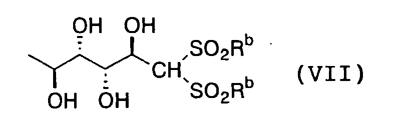 wherein R^{b} is defined as the same as above;
and, treating in base the obtained disulfonyl derivative represented by formula (VII) to prepare 5-deoxy-L-arabinose represented by the following formula (VIII).

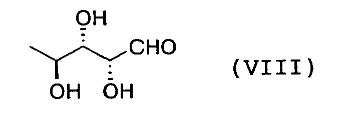

7. A process for producing 5-deoxy-L-arabinose, the process being **characterized by** comprising:
subjecting a dialkylmercaptal compound represented by the following formula (VI) to an oxidation reaction: 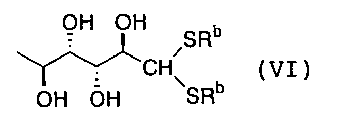
wherein R^{b} represents a C₁₁₋₁₆ straight chain alkyl group, to prepare a disulfonyl derivative represented by the following formula (VII): 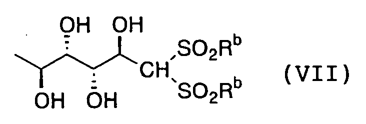 wherein R^{b} is defined as the same as above;
and, treating in base the obtained disulfonyl derivative represented by formula (VII) to prepare 5-deoxy-L-arabinose represented by the following formula (VIII).

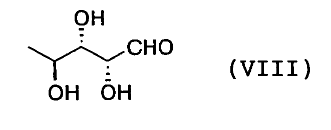

8. The process for producing 5-deoxy-L-arabinose according to claim 6 or 7, wherein as the oxidant in the oxidation reaction, hydrogen peroxide, peracetic acid, m-chloroperbenzoic acid or oxone is used.

9. A process for producing 5-deoxy-L-arabinose, the process being **characterized by** comprising:
treating in base a disulfonyl derivative represented by the following formula (VII): 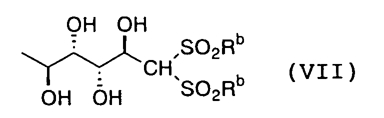
wherein R^{b} represents a C₁₁₋₁₆ straight chain alkyl group, to prepare 5-deoxy-L-arabinose represented by the following formula (VIII).

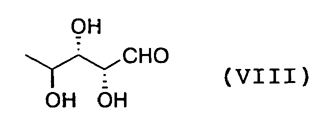

10. The production process according to any one of claims 1 to 9, wherein R^{b} in the formulas is a C₁₂ dodecyl group.

11. A dialkylmercaptal compound represented by the following formula (VI): 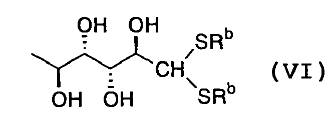 wherein R^{b} represents a C₁₁₋₁₆ straight chain alkyl group.

12. A disulfonyl derivative represented by the following formula (VII): 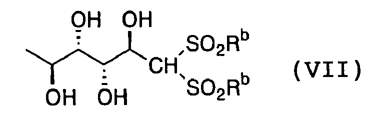 wherein R^{b} represents a C₁₁₋₁₆ straight chain alkyl group.

13. The compound according to claim 11 or 12, wherein R^{b} in the formulas is a C₁₂ alkyl group.
